(19) Europäisches Patentamt
European Patent Office
Office européen des brevets

(11) **EP 3 200 602 B1**

(12) **EUROPÄISCHE PATENTSCHRIFT**

(45) Veröffentlichungstag und Bekanntmachung des Hinweises auf die Patenterteilung:
**10.03.2021 Patentblatt 2021/10**

(51) Int Cl.:
*A23K 10/00* (2016.01)   *A23K 10/40* (2016.01)
*A23K 50/00* (2016.01)   *A61K 31/202* (2006.01)
*C12P 7/64* (2006.01)   *A61K 35/66* (2015.01)
*A23K 10/12* (2016.01)   *A23K 20/158* (2016.01)
*A23K 20/163* (2016.01)   *A23K 50/80* (2016.01)
*A23K 40/25* (2016.01)

(21) Anmeldenummer: **15766504.3**

(22) Anmeldetag: **22.09.2015**

(86) Internationale Anmeldenummer:
**PCT/EP2015/071635**

(87) Internationale Veröffentlichungsnummer:
**WO 2016/050552 (07.04.2016 Gazette 2016/14)**

(54) **PUFA ENTHALTENDE BIOMASSE MIT HOHER ZELLSTABILITÄT UND DEREN VERWENDUNG ZUR HERSTELLUNG VON FUTTERMITTELN**

PUFA-CONTAINING BIOMASS WITH HIGH CELL STABILITY AND ITS USE IN THE PRODUCTION OF ANIMAL FEED

BIOMASSE CONTENANT DES PUFAS ET PRÉSENTANT UNE GRANDE STABILITÉ CELLULAIRE, ET SON UTILISATION DANS LA PRODUCTION D'ALIMENTS POUR ANIMAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2014 EP 14187471**

(43) Veröffentlichungstag der Anmeldung:
**09.08.2017 Patentblatt 2017/32**

(73) Patentinhaber: **Evonik Operations GmbH**
**45128 Essen (DE)**

(72) Erfinder:
• **PRIEFERT, Horst**
**48346 Ostbevern (DE)**
• **SCHNEIDER, Jens**
**33615 Bielefeld (DE)**
• **RABE, Christian**
**63762 Großostheim (DE)**
• **SILVA, Amelia Claudia**
**63457 Hanau (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-01/54510       WO-A1-94/08467**
**WO-A1-2014/122092    WO-A2-97/37032**

• **"Taxonomy browser (Aurantiochytrium limacinum)", , 19. Januar 2015 (2015-01-19), XP055163221, Gefunden im Internet: URL:http://www.ncbi.nlm.nih.gov/Taxonomy/Browser/wwwtax.cgi?mode=Info&id=87102&lvl=3 &lin=f&keep=1&srchmode=1&unlock [gefunden am 2015-01-19]**

• **Rajagopalan Prabu ET AL: "Effect of sodium sulphate salinity for production of docosahexaenoic acid (DHA) by Thraustochytrids aureum RAK-21", Asian Biomedicine, Oktober 2012 (2012-10), Seiten 693-701, XP055163274, DOI: 10.5372/1905-7415.0605.109 Gefunden im Internet: URL:http://thailand.digitaljournals.org/in dex.php/ABM/article/view/13783 [gefunden am 2015-01-20]**

Bemerkungen:

Die Akte enthält technische Angaben, die nach dem Eingang der Anmeldung eingereicht wurden und die nicht in dieser Patentschrift enthalten sind.

**Beschreibung**

Die vorliegende Erfindung betrifft eine PUFAs enthaltende Biomasse mit hoher Zellstabilität.

Verfahren zur Herstellung von polyungesättigte Fettsäuren (PUFAs) enthaltender Biomasse sind im Stand der Technik bereits beschrieben. Die WO05154510 beschreibt ein Verfahren zur Anzucht von *Schizochytrium sp.* in einem Fermentationsmedium, das Sulfat enthält. Verschiedene Biomassedichten werden erreicht. Ein Problem bei der erhaltenen Biomasse stellt häufig die Stabilität der Zellwand dar. Die Zellwand darf nicht zu labil sein, da ansonsten das enthaltene Öl zu früh freigesetzt und die ungesättigten Fettsäuren hierbei oxidiert werden könnten. Andererseits darf die Zellwand auch nicht zu stabil sein, da ansonsten bei der Herstellung des Futtermittels die Zellen nicht vollständig aufgebrochen werden und/oder zum Aufbrechen der Zellen ein sehr hoher Energieeintrag erforderlich ist, so dass das Öl nicht vollständig oder nur unter sehr hohem Energieeintrag und damit hohen Kosten aus den Zellen freigesetzt werden kann.

Aufgabe der vorliegenden Erfindung war es daher, eine Biomasse zur Verfügung zu stellen, die eine für die Weiterverarbeitung zum Futtermittel geeignete Zellstabilität aufweist, die weder zu hoch noch zu niedrig sein darf.

Überraschenderweise wurde gefunden, dass durch gezielte Zugabe von Sulfat zum Fermentationsmedium von Zellen des Taxons Labyrinthulomycetes die Stabilität der Zellwand optimiert werden kann.

Es hat sich hierbei herausgestellt, dass durch Zugabe von Sulfat in einer Menge, so dass sich in der resultierenden Biomasse eine Sulfat-Konzentration von 25 bis 40 g/kg einstellt, eine optimale Zellstabilität erreicht werden kann.

Außerdem hat sich gezeigt, dass die so erhaltene Biomasse bei einem sehr niedrigen Energieeintrag zu einem Futtermittel mit hoher Abriebfestigkeit und hoher Wasserstabilität weiterverarbeitet werden kann.

Weiterhin hat sich gezeigt, dass das unter Verwendung der erfindungsgemäßen Biomasse erhaltene Futtermittel besonders vorteilhaft zur Anzucht von Fischen eingesetzt werden kann.

Eine weitere Aufgabe der vorliegenden Erfindung kann daher darin gesehen werden, eine Biomasse bereitzustellen, die sich aufgrund ihrer Eigenschaften in besonders gutem Maße dazu eignet, zu einem Futtermittel weiterverarbeitet werden zu können.

Gegenstand der vorliegenden Erfindung ist daher eine PUFAs enthaltende Biomasse, die einen Sulfat-Gehalt von 25 bis 40 g/kg, bezogen auf die Trockenmasse, sowie einen Feuchtigkeitsgehalt von unter 5 Gew.-% aufweist, wobei die Biomasse Zellen des Taxons Labyrinthulomycetes enthält. Der Sulfat-Gehalt beträgt vorzugsweise 25 bis 35 g/kg, bezogen auf die Trockenmasse.

Unter "Sulfat-Gehalt" ist erfindungsgemäß der Gesamtgehalt an Sulfat, also der Gehalt an freiem und gebundenem, insbesondere organisch gebundenem, Sulfat zu verstehen. Es ist davon auszugehen, dass der Großteil des in der Biomasse enthaltenen Sulfats als Bestandteil von Exopolysacchariden vorliegt, die an der Ausbildung der Zellwand der Mikroorganismen beteiligt sind.

Die Bestimmung des Sulfat-Gehalts erfolgt durch Ermittlung des Schwefel-Gehalts der erhaltenen Biomasse, da der Großteil des in der Biomasse enthaltenen Schwefels auf das enthaltene Sulfat zurückzuführen ist. Schwefel, der auf andere Quellen zurückzuführen ist, ist aufgrund der Menge des enthaltenen Sulfats zu vernachlässigen. Aus der Menge des ermittelten Schwefels kann somit ohne Weiteres die Menge an enthaltenem Sulfat ermittelt werden.

Der Schwefel-Gehalt der Biomasse wird hierbei durch Elementaranalyse gemäß DIN EN ISO 11885 bestimmt. Für die Analyse des Schwefelgehalts der Biomasse werden entsprechende Aliquote der Probe vor der Analyse vorzugsweise mit Salpetersäure und Wasserstoffperoxid bei 240°C unter Druck aufgeschlossen, um die freie Zugänglichkeit des enthaltenen Schwefels sicherzustellen.

Der Phosphor-Gehalt der Biomassen beträgt, in Bezug auf die Trockenmasse, vorzugsweise 1 bis 6 g/kg, insbesondere 2 bis 5 g/kg. Der Phosphor-Gehalt wird vorzugsweise ebenfalls durch Elementaranalyse gemäß DIN EN ISO 11885 ermittelt.

Eine erfindungsgemäße Biomasse gemäß Anspruch 1 umfasst Zellen, und besteht vorzugsweise im Wesentlichen aus solchen Zellen, des Taxons Labyrinthulomycetes (Labyrinthulea, Netzschleimpilze, Schleimnetze), insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium, Aurantiochytrium, Oblongichytrium und Ulkenia. Besonders bevorzugt handelt es sich um Zellen der Gattungen Thraustochytrium, Schizochytrium, Aurantiochytrium oder Oblongichytrium, vor allem um solche der Gattung Aurantiochytrium. Ein besonders bevorzugter Stamm stellt der Stamm Aurantiochytrium limacinum SR21 (IFO 32693) dar.

Bei der erfindungsgemäßen Biomasse handelt es sich vorzugsweise um das Produkt eines fermentativen Kultivierungsverfahrens. Die Biomasse kann entsprechend neben den aufzuschließenden Zellen auch noch Bestandteile des Fermentationsmediums enthalten. Bei diesen Bestandteilen kann es sich insbesondere um Salze, Antischaummittel und nicht umgesetzte Kohlenstoffquelle und/oder Stickstoffquelle handeln. Der Zellgehalt in dieser Biomasse beträgt vorzugsweise mindestens 70 Gew.-%, vorzugsweise mindestens 75 Gew.-%. Der Zellgehalt in der Biomasse kann gegebenenfalls vor Durchführung des Zellaufschlussverfahrens durch entsprechende Waschschritte beispielsweise auf mindestens 80 oder mindestens 90 Gew.-% erhöht werden. Die erhaltene Biomasse kann jedoch auch direkt in dem Zellaufschlussverfahren eingesetzt werden.

Die Zellen der Biomasse zeichnen sich vorzugsweise dadurch aus, dass sie einen PUFA-Gehalt von mindestens 20 Gew.-%, vorzugsweise mindestens 30 Gew.-%, insbesondere mindestens 35 Gew.-%, jeweils bezogen auf die Zelltrockenmasse, aufweisen.

In einer bevorzugten Ausführungsform liegt ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Vorzugsweise handelt es sich bei mindestens 10 Gew.-%, insbesondere mindestens 20 Gew.-%, besonders bevorzugt 20 bis 60 Gew.-%, insbesondere 20 bis 40 Gew.-%, der in der Zelle enthaltenen Fettsäuren um PUFAs.

Unter polyungesättigten Fettsäuren (PUFAs) werden erfindungsgemäß Fettsäuren verstanden, die mindestens zwei C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Verfahren zur Herstellung der PUFAs enthaltenden Zellen insbesondere der Ordnung Thraustochytriales sind im Stand der Technik ausführlich beschrieben (siehe z.B. WO91/07498, WO94/08467, WO97/37032, WO97/36996, WO01/54510). Ein Herstellungsverfahren für die PUFAs enthaltende Biomasse ist nicht Teil der vorliegenden Erfindung und wird nachstehend nur zu Illustrationszwecken beschrieben. Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden. Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Die Erzielung des erwünschten Sulfat-Gehalts in der resultierenden Biomasse kann auf unterschiedliche Weise erfolgen.

Beispielsweise kann in einem sogenannten Batch-Verfahren die benötigte Menge an Sulfat bereits zu Beginn vollständig vorgelegt werden. Die Menge an benötigtem Sulfat lässt sich einfach berechnen, da die zur Bildung der Biomasse eingesetzten Zellen das Sulfat fast vollständig assimilieren.

Bei Anwendung eines sogenannten Fed-Batch-Verfahrens kann alternativ die Menge an benötigten Sulfat im Verlauf der Fermentation zudosiert werden oder entsprechend ein Teil des Sulfats vorgelegt und der Rest im Verlauf der Fermentation zudosiert werden. Insbesondere dann, wenn sich im Verlauf der Fermentation herausstellt, dass die Menge an produzierter Biomasse den ursprünglich kalkulierten Wert übertrifft, kann durch nachträgliches Zudosieren von Sulfat sichergestellt werden, dass die resultierende Biomasse über ausreichende Zellstabilität verfügt.

Als Sulfat-Salz wird vorzugsweise Natriumsulfat, Ammoniumsulfat oder Magnesiumsulfat sowie Mischungen davon eingesetzt.

Der Chlorid-Gehalt liegt während der Fermentation, in Bezug auf das flüssige Fermentationsmedium einschließlich der enthaltenen Biomasse, vorzugsweise stets unter 3 g/kg, insbesondere unter 1 g/kg, besonders bevorzugt unter 400 mg/kg Fermentationsmedium.

Neben Sulfaten und gegebenenfalls eingesetzten Chloriden können bei der Fermentation gegebenenfalls auch weitere Salze, insbesondere ausgewählt aus Natriumcarbonat, Natriumhydrogencarbonat, Sodaasche oder anorganischen Phosphorverbindungen, eingesetzt werden.

Sofern weitere Salze eingesetzt werden, werden diese vorzugsweise in einer Menge eingesetzt, so dass jedes einzelne während der Fermentation, in Bezug auf das flüssige Fermentationsmedium einschließlich der enthaltenen Biomasse, jeweils in einer Menge von stets weniger als 10 g/kg, insbesondere weniger als 5 g/kg, besonders bevorzugt weniger als 3 g/kg im Fermentationsmedium vorliegt.

Der Gesamtsalzgehalt im Fermentationsmedium einschließlich der enthaltenen Biomasse liegt vorzugsweise im Verlauf der gesamten Fermentation stets unter 35 g/kg, insbesondere unter 30 g/kg. Besonders bevorzugt liegt der Gesamtsalzgehalt während der gesamten Fermentation, in Bezug auf das flüssige Fermentationsmedium einschließlich der enthaltenen Biomasse, zwischen 10 und 35 g/kg, insbesondere zwischen 12 und 30 g/kg.

Der Sulfat-Gehalt im Fermentationsmedium einschließlich der enthaltenen Biomasse liegt vorzugsweise im Verlauf der gesamten Fermentation stets zwischen 5 und 16 g/kg.

Zusätzlich können in das Fermentationsmedium auch organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Die Fermentation der Zellen erfolgt vorzugsweise bei einem pH-Wert von 3 bis 11, insbesondere 4 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Die Zellen werden vorzugsweise bis zu einer Biomassendichte von mindestens 50, 60 oder 70 g/l, insbesondere mindestens 80 oder 90 g/l, besonders bevorzugt mindestens 100 g/l, fermentiert. Die Angaben beziehen sich hierbei auf den Gehalt an Bio-Trockenmasse in Bezug auf das Gesamtvolumen der Fermentationsbrühe nach Beendigung der Fermentation. Der Gehalt an Bio-Trockenmasse wird bestimmt durch Abfiltrieren der Biomasse aus der Fermentationsbrühe, anschließendes Waschen mit Wasser, danach vollständige Trocknung - beispielsweise in der Mikrowelle - und schließlich Feststellung des Trockengewichts.

Vorzugsweise erfolgt nach der Ernte der Zellen oder gegebenenfalls auch schon kurz vor der Ernte der Zellen eine Pasteurisierung der Zellen, um die Zellen abzutöten und Enzyme, die den Abbau der Lipide befördern könnten, zu inaktivieren.

Nach Beendigung der Fermentation erfolgt die Ernte der Biomasse. Durch Zentrifugation, Filtration, Dekantieren oder Lösungsmittelverdampfung kann ein Großteil des Fermentationsmediums von der Biomasse abgetrennt werden. Die Lösungsmittelverdampfung erfolgt vorzugsweise unter Einsatz eines Trommeltrockners, eines Tunneltrockners, durch Sprühtrocknung oder Vakuumverdampfung. Die Lösungsmittelverdampfung kann insbesondere auch unter Einsatz eines Rotationsverdampfers, eines Dünnschichtverdampfers oder eines Fallfilmverdampfers erfolgen. Alternativ zur Lösungsmittelverdampfung kommt beispielsweise auch die Umkehrosmose zur Einengung der Fermentationsbrühe in Betracht. Anschließend wird die erhaltene Biomasse weiter getrocknet, vorzugsweise durch Fließbettgranulation. Durch das Trocknen wird der Feuchtigkeitsgehalt auf unter 5 Gew.-% reduziert.

Unter "Trockenmasse" ist erfindungsgemäß entsprechend eine Biomasse zu verstehen, die einen Feuchtigkeitsgehalt von unter 5 Gew.-% aufweist.

Die Trocknung der Biomasse erfolgt in einer besonders bevorzugten Ausführungsform in einem Wirbelschichtgranulationsverfahren oder einem Düsensprühtrocknungsverfahren, wie beispielsweise in EP13176661.0 beschrieben.

Der Biomasse kann während der Trocknung gegebenenfalls Kieselsäure als Antibackmittel zugesetzt werden, um sie in einen besser handhabbaren Zustand zu überführen. Die Biomasse enthaltende Fermentationsbrühe sowie die Kieselsäure werden hierzu vorzugsweise in die jeweilige Trocknungszone eingesprüht. Alternativ wird die Biomasse vorzugsweise erst nach der Trocknung mit der Kieselsäure vermischt. Es wird diesbezüglich insbesondere auch auf die Patentanmeldung EP13187631.0 verwiesen.

In einer bevorzugten Ausführungsform weist eine erfindungsgemäß einzusetzende Biomasse nach der Trocknung eine Konzentration an Kieselsäure, insbesondere hydrophiler oder hydrophober Kieselsäure, von 0,2 bis 10 Gew.-%, insbesondere 0,5 bis 5 Gew.-%, vor allem 0,5 bis 2 Gew.-%, auf.

Durch die Trocknung wird vorzugweise ein rieselfähiges, feinteiliges oder grobkörniges Produkt, vorzugsweise Granulat, erhalten. Gegebenenfalls kann aus dem erhaltenen Granulat durch Sieben oder Staubabtrennung ein Produkt mit der gewünschten Korngröße erhalten werden.

Sofern ein rieselfähiges feinteiliges Pulver erhalten wurde, kann dieses gegebenenfalls durch geeignete Kompaktier- oder Granulierverfahren in ein grobkörniges, gut rieselfähiges, lagerbares und weitgehend staubfreies Produkt überführt werden.

Bei dieser anschließenden Granulation oder Kompaktierung können gegebenenfalls übliche organische oder anorganische Hilfsstoffe beziehungsweise Träger wie Stärke, Gelatine, Cellulosederivate oder ähnliche Stoffe, die üblicherweise in der Lebensmittel- oder Futterverarbeitung als Binde-, Gelier- oder Verdickungsmittel Verwendung finden, eingesetzt werden.

Unter "rieselfähig" ist erfindungsgemäß ein Pulver zu verstehen, das aus einer Serie von Glasauslaufgefäßen mit verschieden großen Auslassöffnungen mindestens aus dem Gefäß mit der Öffnung 5 Millimeter ungehindert auslaufen kann (Klein: Seifen, Öle, Fette, Wachse 94, 12 (1968)).

Unter "feinteilig" ist ein Pulver mit einem überwiegenden Anteil (> 50 %) einer Korngröße von 20 bis 100 Mikrometer Durchmesser zu verstehen.

Unter "grobkörnig" ist ein Pulver mit einem überwiegenden Anteil (>50 %) einer Korngröße von 100 bis 2500 Mikrometer Durchmesser zu verstehen.

Unter "staubfrei" ist ein Pulver zu verstehen, das lediglich geringe Anteile (< 10 %, vorzugsweise < 5 %) an Korngrößen unter 100 Mikrometer enthält.

Die Bestimmung der Korn- bzw. Teilchengrößen erfolgt vorzugsweise durch Methoden der Laserbeugungsspektrometrie. Die anzuwendenden Methoden sind im Lehrbuch "Teilchengrößenmessung in der Laborpraxis" von R.H. Müller und R. Schuhmann, Wissenschaftliche Verlagsgesellschaft Stuttgart (1996) sowie im Lehrbuch "Introduction to Particle Technology" von M. Rhodes, Verlag Wiley & Sons (1998) beschrieben. Sofern unterschiedliche Methoden anwendbar sind, wird bevorzugt die zuerst genannte anwendbare Methode aus dem Lehrbuch von R.H. Müller und R. Schuhmann zur Teilchengrößenmessung angewendet.

Die durch Trocknungsverfahren erhaltenen Produkte besitzen vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 3500 Mikrometern, vorzugsweise 100 bis 3000 Mikrometern, vor allem 100 bis 2500 Mikrometern.

Die erhaltenen Produkte eines Wirbelschichtgranulationsverfahrens besitzen hierbei vorzugsweise einen Anteil von mindestens 80 Gew.-%, insbesondere mindestens 90 Gew.-%, besonders bevorzugt mindestens 95 Gew.-%, an Partikeln mit einer Korngröße von 200 bis 3500 Mikrometern, vorzugsweise 300 bis 3000 Mikrometern, vor allem 500 bis 2500 Mikrometern.

Die erhaltenen Produkte eines Sprühtrocknungsverfahrens besitzen hingegen vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 500 Mikrometern, vorzugsweise 100 bis 400 Mikrometern, vor allem 100 bis 300 Mikrometern.

Die erhaltenen Produkte eines Sprühtrocknungsverfahrens und anschließenden Granulationsverfahrens besitzen vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 1000 Mikrometern.

Der Anteil an Staub, d.h. Teilchen mit einer Korngröße von kleiner 100 Mikrometern, liegt vorzugsweise bei maximal 10 Gew.-%, insbesondere maximal 8 Gew.-%, besonders bevorzugt maximal 5 Gew.-%, vor allem maximal 3 Gew.-%.

Das Schüttgewicht der erfindungsgemäßen Produkte liegt vorzugsweise bei 400 bis 800 kg/m$^3$, besonders bevorzugt bei 450 bis 700 kg/m$^3$.

Erfindungsgemäß hat sich weiterhin herausgestellt, dass sich eine erfindungsgemäße Biomasse bei niedrigem Energieeintrag zu einem Futtermittel mit hoher Ölbeladungskapazität, hoher Abriebfestigkeit und hoher Wasserstabilität weiterverarbeiten lässt.

Ein weiterer Gegenstand der vorliegenden Erfindung ist daher auch ein Futtermittel, das eine erfindungsgemäße Biomasse sowie weitere Futtermittelinhaltsstoffe umfasst.

Die weiteren Futtermittelinhaltsstoffe sind hierbei vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Ein erfindungsgemäßes Futtermittel bzw. eine zur Herstellung eines erfindungsgemäßen Futtermittels verwendete Zusammensetzung zeichnet sich erfindungsgemäß vorzugsweise dadurch aus, dass es eine erfindungsgemäße Biomasse in einer Menge von 2 bis 24 Gew.-%, vorzugsweise 4 bis 22 Gew.-%, insbesondere 9 bis 20 Gew.-%, vor allem 11 bis 18 Gew.-%, enthält.

Die zur Herstellung des Futtermittels verwendete Zusammensetzung weist bevorzugt folgende Eigenschaften auf:

a) einen Gesamt-Proteingehalt von 33 bis 67 Gew.-%, vorzugsweise 39 bis 61 Gew.-%, insbesondere 44 bis 55 Gew.-%,

b) einen Gesamt-Fettgehalt von 5 bis 25 Gew.-%, vorzugsweise 8 bis 22 Gew.-%, insbesondere 10 bis 20 Gew.-%, vor allem 12 bis 18 Gew.-%;

c) einen Gesamt-Stärkegehalt von maximal 25 Gew.-%, insbesondere maximal 20 Gew.-%, vorzugsweise 6 bis 17 Gew.-%, besonders bevorzugt 8 bis 14 Gew.-%;

d) einen Gehalt an erfindungsgemäßer Biomasse, insbesondere einer erfindungsgemäßen Labyrinthulea-Biomasse, bevorzugt einer erfindungsgemäßen Thraustochytriaceae-Biomasse, von 2 bis 24 Gew.-%, vorzugsweise 4 bis 22 Gew.-%, insbesondere 9 bis 20 Gew.-%, vor allem 11 bis 18 Gew.-%;

e) vorzugsweise einen Gehalt an polyungesättigten Fettsäuren (PUFAs) von 0,8 bis 8 Gew.-%, vorzugsweise 1,2 bis 6 Gew.-%, insbesondere 1,4 bis 5 Gew.-%, vor allem 1,5 bis 4 Gew.-%;

f) vorzugsweise einen Gehalt an omega-3-Fettsäuren von 0,8 bis 8 Gew.-%, vorzugsweise 1,2 bis 6 Gew.-%, insbesondere 1,4 bis 5 Gew.-%, vor allem 1,5 bis 4 Gew.-%;

g) vorzugsweise Gehalt an DHA von 0,1 bis 4,0 Gew.-%, vorzugsweise 0,25 bis 3,0 Gew.-%, insbesondere 0,5 bis 2,8 Gew.-%, vor allem 0,8 bis 2,5 Gew.-%, insbesondere 1,0 bis 2,0 Gew.-%.

Durch Extrusion der zuvor genannten Zusammensetzungen lässt sich ein Extrudat mit einer Abriebfestigkeit von mindestens 91 %, insbesondere mindestens 92, 93 oder 94 % erhalten.

Diese Extrudate stellen einen bevorzugten Gegenstand der vorliegenden Erfindung dar.

Die Bestimmung der Abriebfestigkeit erfolgte erfindungsgemäß auf folgende Weise: Das getrocknete Extrudat (mit 4 mm Durchmesser und 4 mm Länge) wurde einer mechanischen Belastung unter Verwendung des Holmen Pellet-Tester NHP100 (Borregaard Lignotech, Hull, UK) ausgesetzt. Vor Durchführung des Tests wurden die Proben gesiebt, um möglicherweise anhaftende Feinteilchen zu entfernen. Die aufbereiteten Proben (100 g) wurden anschließend in den Pellet-Tester eingeführt unter Verwendung eines 2,5 mm-Filtersiebs. Die Pellets wurden anschließend mit hoher Luftgeschwindigkeit (ca. 70 mbar) für 30 Sekunden durch ein Röhrchen mit rechtwinkligen Rohrbogen befördert. Die Versuchsparameter sind gerätebedingt vorgegeben. Anschließend wurde der Abrieb durch Wiegen bestimmt. Die Abriebfestigkeit wurde als PDI (Pellet Durability Index) angegeben, definiert als die Menge an in dem Filtersieb nach Durchführung des Tests verbliebener Probe in Prozent. Der Test wurde jeweils mit drei Proben durchgeführt und anschließend der Mittelwert gebildet.

Es hat sich als erfindungsgemäß besonders vorteilhaft herausgestellt, wenn die Extrusion bei einem Energieeintrag von 12 - 28 Wh/kg, insbesondere 14 - 26 Wh/kg, besonders bevorzugt 16 - 24 Wh/kg, vor allem 18 - 22 Wh/kg, erfolgt.

Im Extrusionsverfahren wird hierbei vorzugsweise ein Schnecken- oder Doppelschneckenextruder eingesetzt. Das Extrusionsverfahren wird vorzugsweise bei einer Temperatur von 80 - 220° C, insbesondere 80 - 130 °C, vor allem 95 - 110°C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Das Extrusionsverfahren kann gegebenenfalls einen Kompaktierungs- und/oder einen Kompressionsschritt umfassen.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken. Die Wasserdampfinjektion wird hierbei vorzugsweise bei einem Druck von 1 bis 5 bar, besonders bevorzugt bei einem Druck von 2 bis 4 bar, durchgeführt.

Die weiteren Nahrungs- oder Futtermittelinhaltsstoffe werden vor dem Vermischen mit der Algen-Biomasse - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Das erzeugte Extrudat hat vorzugsweise einen Durchmesser von 1 bis 14 mm, vorzugsweise 2 bis 12 mm, insbesondere 2 bis 6 mm, und weist vorzugsweise auch eine Länge von 1 bis 14 mm, vorzugsweise 2 bis 12 mm, insbesondere 2 bis 6 mm, auf. Die Länge des Extrudats wird durch Einsatz eines Schneidwerkzeugs während der Extrusion eingestellt. Die Länge des Extrudats wird vorzugsweise so gewählt, dass es dem Durchmesser des Extrudats in etwa entspricht. Der Durchmesser des Extrudats wird durch Wahl des Siebdurchmessers festgelegt.

In einer erfindungsgemäß bevorzugten Ausführungsform schließt sich an das Extrusionsverfahren die Beladung des erhaltenen Extrudats mit Öl an. Hierzu wird das Extrudat vorzugsweise zunächst auf einen Feuchtigkeitsgehalt von maximal 5 Gew.-% getrocknet. Die Beladung des Extrusionsprodukts mit Öl kann erfindungsgemäß beispielsweise durch Einlegen des Extrudats in Öl oder Besprühen des Extrudats mit Öl erfolgen, sie erfolgt erfindungsgemäß jedoch vorzugsweise durch Vakuumbeschichtung.

Auf diese Weise werden Futtermittel erhalten, die erfindungsgemäße Biomassen vorzugsweise in einer Menge von 2 bis 22 Gew.-%, insbesondere 4 bis 20 Gew.-%, besonders bevorzugt 8 bis 18 Gew.-%, vor allem 10 bis 16 Gew.-%, enthalten.

Diese Futtermittel weisen entsprechend vorzugsweise weiterhin mindestens eine, vorzugsweise alle, der folgenden Eigenschaften auf:

a) einen Gesamt-Proteingehalt von 30 bis 60 Gew.-%, vorzugsweise 35 bis 55 Gew.-%, insbesondere 40 bis 50 Gew.-%;

b) einen Gesamt-Fettgehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

a) einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;

b) einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

c) einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

Ein erfindungsgemäß bevorzugter Gegenstand ist daher auch ein Futtermittel, insbesondere ein Extrudat, das über mindestens eine, vorzugsweise alle, der folgenden Eigenschaften verfügt:

a) einen Gesamt-Proteingehalt von 30 bis 60 Gew.-%, vorzugsweise 35 bis 55 Gew.-%, insbesondere 40 bis 50 Gew.-%;

b) einen Gesamt-Fettgehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

d) einen Gehalt an einer erfindungsgemäßen Biomasse, insbesondere einer erfindungsgemäßen Labyrinthulea-Biomasse, vorzugsweise einer erfindungsgemäßen Thraustochytriaceae-Biomasse, von 2 bis 22 Gew.-%, vorzugsweise 4 bis 20 Gew.-%, insbesondere 8 bis 18 Gew.-%, vor allem 10 bis 16 Gew.-%;

e) einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;

f) einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

g) einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

Ein erfindungsgemäß bevorzugter Gegenstand ist daher auch ein Futtermittel, insbesondere ein Extrudat, das über mindestens eine, vorzugsweise alle, der folgenden Eigenschaften verfügt:

a) einen Gesamt-Protein-Gehalt von 30 bis 60 Gew.-%, vorzugsweise 35 bis 55 Gew.-%, insbesondere 40 bis 50 Gew.-%;

b) einen Gesamt-Fett-Gehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

h) einen Gehalt an einer erfindungsgemäßen Aurantiochytrien-Biomasse, bevorzugt einer erfindungsgemäßen Aurantiochytrium limacinum-Biomasse, vor allem einer erfindungsgemäßen Aurantiochytrium limacinum SR21-Biomasse, von 2 bis 22 Gew.-%, vorzugsweise 4 bis 20 Gew.-%, insbesondere 8 bis 18 Gew.-%, vor allem 10 bis 16 Gew.-%;

d) einen Gehalt an polyungesättigten Fettsäuren (PUFAs) von 2 bis 12 Gew.-%, vorzugsweise 3 bis 10 Gew.-%,

insbesondere 4 bis 9 Gew.-%, vor allem 5 bis 8 Gew.-%;

e) einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

f) einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

Die zuvor genannten, durch Ölbeschichtung erhältlichen Extrudate besitzen vorzugsweise eine Wasserstabilität von mindestens 96 %, insbesondere mindestens 97 oder 98 %, und stellen einen weiteren bevorzugten Gegenstand der vorliegenden Erfindung dar.

Die Bestimmung der Wasserstabilität wurde im Wesentlichen ausgeführt wie durch Baeverfjord et al. (2006; Aquaculture 261, 1335-1345) beschrieben, mit geringen Modifikationen. 10 g-Proben des Extrudats (mit einer Länge und einem Durchmesser von jeweils 4 mm) wurden in metallische Infusions-Körbchen (Inox, Deutschland) mit einem Durchmesser von 6,5 mm und einer Maschenweite von 0,3 mm gegeben. Die Infusions-Körbchen wurden anschließend in eine Plastik-Wanne mit Wasser gegeben, so dass die Proben vollständig mit Wasser bedeckt waren. Die Wanne wurde anschließend für 30 Minuten einer Schüttelagitation von 30 Schütteleinheiten pro Minute unter Verwendung des Multiorbital-Schüttlers PSU-20I (Biosan, Lettland) ausgesetzt. Danach wurden die Proben vorsichtig mit Löschpapier getrocknet und anschließend gewogen bevor und nachdem sie einer Ofen-Trocknung bei einer Temperatur von 105 °C für 24 Stunden unterzogen worden waren. Die Wasserstabilität wurde berechnet als die Differenz des Trockengewichts der Probe vor und nach der Inkubation in Wasser und in Prozent des Trockengewichts der eingesetzten Probe vor der Inkubation mit Wasser angegeben.

Als fetthaltige Komponente können erfindungsgemäß neben der erfindungsgemäß einzusetzenden Biomasse Fette, insbesondere Öle, tierischen als auch solche pflanzlichen Ursprungs eingesetzt werden. Als fetthaltige Komponente kommen erfindungsgemäß insbesondere Pflanzenöle in Betracht, beispielsweise Sojabohnenöl, Rapssamenöl, Sonnenblumenkernöl, Flachssamenöl oder Palmöl sowie Mischungen davon. Daneben kann gegebenenfalls auch Fischöl als fetthaltige Komponente in geringen Mengen eingesetzt werden.

Vorzugsweise enthält ein erfindungsgemäßes Futtermittel, das eine Abriebfestigkeit von mindestens 96, 97 oder 98 % aufweist, pflanzliche Öle in einer Menge von 3 bis 18 Gew.-%, insbesondere 5 bis 15 Gew.-%, vor allem 7 bis 13 Gew.-%. Wie zuvor beschrieben wird das pflanzliche Öl hierbei vorzugsweise nachträglich auf das Extrudat aufgetragen, insbesondere durch Vakuumbeschichtung.

Als proteinhaltige Komponente können erfindungsgemäß beispielsweise Sojaprotein, Erbsenprotein, Weizengluten oder Maisgluten sowie Mischungen davon eingesetzt werden.

Als proteinhaltige Komponente, die zusätzlich Fette enthält, können beispielsweise eingesetzt werden Fischmehl, Krillmehl, Muschelmehl, Kalmarmehl oder Schrimpsschalen. Diese werden im Folgenden unter dem Begriff "marines Mehl" zusammengefasst. In einer bevorzugten Ausführungsform umfasst ein erfindungsgemäßes Futtermittel marines Mehl, vorzugsweise Fischmehl, in einer Menge von 3 bis 18 Gew.-%, insbesondere 5 bis 15 Gew.-%, vor allem 7 bis 13 Gew.-%.

Als kohlenhydrathaltige Komponente können beispielsweise Weizenmehl, Sonnenblumenmehl oder Sojamehl sowie Mischungen davon eingesetzt werden.

Beim Einsatz erfindungsgemäßer Futtermittel, insbesondere eines erfindungsgemäßen ölbeschichteten Extrudats, in der Tierzucht, hat sich herausgestellt, dass dieses in besonderem Maße das Wachstum der Tiere fördert sowie den Stress-Zustand der Tiere verbessert.

Ein weiterer Gegenstand der vorliegenden Erfindung ist auch ein Verfahren zur Anzucht von Tieren, dadurch gekennzeichnet, dass diesen ein erfindungsgemäßes Futtermittel verabreicht wird.

Verzugsweise wird in dem erfindungsgemäßen Verfahren das Futtermittel mindestens alle zwei Tage, vorzugsweise mindestens einmal täglich, verabreicht.

Bei den angezüchteten Tieren, die mit einem erfindungsgemäßen Futtermittel gefüttert werden, handelt es sich vorzugsweise um Geflügel, Schweine oder Rinder.

Besonders bevorzugt handelt es sich bei den angezüchteten Tieren jedoch um marine Tiere, besonders bevorzugt um Flossenfische oder Crustaceen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich bei den angezüchteten Tieren um Lachse. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Bei den angezüchteten Tieren kann es sich insbesondere auch um Fische handeln, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Es kann sich bei den angezüchteten Tieren jedoch auch um Kleinlebewesen handeln, die in der Aquakultur als Futtermittel eingesetzt werden. Bei diesen Kleinlebewesen kann es sich beispielsweise um Nematoden, Crustaceen oder Rotiferen handeln.

Die Anzucht der marinen Tiere kann in Teichen, Tanks, Becken oder auch in abgegrenzten Arealen im Meer oder in Seen, hierbei insbesondere in Käfigen oder Netzpferchen, erfolgen. Die Anzucht kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

Bei dem erfindungsgemäßen Futtermittel handelt es sich entsprechend vorzugsweise um ein Futtermittel zum Einsatz bei der Anzucht der zuvor genannten Tiere.

**Ausführungsbeispiele**

*Beispiel 1: Herstellung der Biomasse durch Fermentation von Aurantiochytrium limacinum SR21 in Medien mit unterschiedlichem Natriumsulfat-Gehalt* (Beispiel nicht Teil der vorliegenden Erfindung und nur zu Illustrationszwecken)

Die Kultivierung der Zellen erfolgte für ca. 75 h in einem Zulaufverfahren unter Verwendung eines Stahlfermenters mit einem Fermentervolumen von 2 Litern bei einer Gesamt-Startmasse von 712 g und einer erreichten Gesamt-Endmasse von 1,3 - 1,5 kg. Während des Verfahrens wurde eine Glucose-Lösung (570 g/kg Glucose) zudosiert ("Fed-batch Verfahren")

Die Zusammensetzung der Startmedien war wie folgt:

Medium 1: 20 g/kg Glukose; 4 g/kg Hefe-Extrakt; 2 g/kg Ammoniumsulfat; 2,46 g/kg Magnesiumsulfat (Heptahydrat); 0,45 g/kg Kaliumchlorid; 4,5 g/kg

Kaliumdihydrogenphosphat; 0,1 g/kg Thiamin (HCl); 5 g/kg Spurenelementlösung.

Medium2: Wie Medium 1 plus 8 g/kg Natriumsulfat

Medium3: Wie Medium 1 plus 12 g/kg Natriumsulfat

Medium4: Wie Medium 1 plus 16 g/kg Natriumsulfat

Die Zusammensetzung der Spurenelementlösung war wie folgt: 35 g/kg Salzsäure (37%); 1,86 g/kg Manganchlorid (Tetrahydrat); 1,82 g/kg Zinksulfat (Heptahydrat); 0,818 g/kg Natrium-EDTA; 0,29 g/kg Borsäure; 0,24 g/kg Natriummolybdat (Dihydrat); 4,58 g/kg Kalziumchlorid (Dihydrat); 17,33 g/kg Eisensulfat (Heptahydrat); 0,15 g/kg Kupferchlorid (Dihydrat).

Die Kultivierung erfolgte unter folgenden Bedingungen: Kultivierungstemperatur 28°C; Belüftungsrate 0,5 vvm, Rührergeschwindigkeit 600 - 1950 rpm, Kontrolle das pH-Werts in der Wachstumsphase bei 4,5 mit Hilfe von Ammoniakwasser (25% v/v). Es wurden folgende Biomasse-Dichten erzielt: 100 g/l (Medium 1), 111 g/l (Medium 2), 114 g/l (Medium 3), 116 g/l (Medium 4).

Nach der Kultivierung wurden die Fermentationsbrühen für 20 Minuten auf 60°C erhitzt um eine weitere Aktivität der Zellen zu unterbinden.

*Beispiel 2: Bestimmung des DHA-Gehalt der Biomassen*

Nach der Inaktivierung wurden die erhaltenen Biomassen einer Fettsäureanalyse unterzogen. Hierzu wurden jeweils 0,2 - 0,5 ml Fermentationsbrühe mit 1ml Internem Standard versehen und mit 9ml einer Methanol/Chloroformlösung (1:2; v/v) aufgefüllt. Die Proben wurden 10 min im Ultraschallbad behandelt. Anschließend wurden die Proben bei 50°C im Thermoblock unter Stickstoffbegasung zur Trockne eingedampft. Zu den Trocknungsrückständen wurden jeweils 2 ml 0,5N KOH geben und für 15 min bei 100°C inkubiert. Anschließend wurden die Proben auf Raumtemperatur abgekühlt, mit jeweils 2 ml 0,7N HCl und 1 ml Bortrifluorid-Lösung (14% BF3 in Methanol) versetzt und weitere 15 min bei 100°C inkubiert. Nach Abkühlen auf Raumtemperatur wurden die Proben jeweils mit einer Mischung aus 3 ml Wasser und 2 ml Heptan extrahiert. Nach Zentrifugation für 1 min bei 2000 U/min wurde jeweils 1 ml der oberen Phase in ein GC-Vial überführt und gaschromatographisch analysiert.

Die Analyse ergab, dass alle vier Biomassen einen DHA-Anteil von mehr als 32 Gew.-% in Bezug auf die Gesamtmenge an enthaltenen Fettsäuren enthielten.

*Beispiel 3: Trocknung der erhaltenen Biomassen*

Die abgekühlten Biomasse-haltigen Fermentationsbrühen mit unterschiedlichen $Na_2SO_4$-Gehalten gemäß Beispiel 1 wurden jeweils separat der Sprühtrocknung unterzogen.

Die Sprühtrocknung erfolgte jeweils unter Verwendung eines Büchi Mini Spray Dryer B-290 (Durchmesser Düsenspitze: 0,7 mm; Durchfluss Sprühluft: 742 L/h; Durchflussrate Aspirator: 35 $m^3$/h; Temperatur Eingangsluft: 220°C; Temperatur Ausgangsluft: 80°C).

*Beispiel 4: Bestimmung der Sulfat- und DHA-Gehalte der sprühgetrockneten Proben*

Die durch Sprühtrocknung der Fermentationsbrühen mit unterschiedlichen Na2SO4-Gehalten erhaltenen Proben wurden einer Sulfat- bzw. Schwefel-Bestimmung und einer DHA- Konzentrationsbestimmung unterzogen. Die DHA-Bestimmung erfolgte wie unter Beispiel 2 beschrieben. Die Bestimmung des Schwefel-Gehalts erfolgte gemäß DIN EN ISO 11885.

Tabelle 1: Analyse sprühgetrockneter Proben

|  | Satzmedium der zur Sprühtrocknung eingesetzten Fermentationsbrühe | | | |
|---|---|---|---|---|
| Eigenschaft | Medium 1 | Medium 2 | Medium 3 | Medium 4 |
| Schwefel nach DIN EN ISO 11885 | 3,60 g/kg | 7,72 g/kg | 10,0 g/kg | 11,0 g/kg |
| DHA Gehalt | 16,6% | 15,6% | 16,0% | 16,0% |

*Beispiel 4: Bestimmung der Verklumpungsneigung*

Die nach Fermentation in Medien mit unterschiedlichen Natriumsulfat-Gehalten erhaltenen Fermentationsbrühen wiesen deutliche Unterschiede im Sprühtrocknungsverfahren auf und das erhaltene sprühgetrocknete Material wies unterschiedlich starke Verklumpungsneigung auf, was auf freigesetztem Öl basierte. Die Biomassen, die durch Fermentation in den Medien 3 und 4 erhalten wurden, zeigten eine deutlich geringere Verklumpungsneigung als die nicht-erfindungsgemäßen Biomassen, die durch Fermentation in den Medien 1 und 2 erhalten wurden. Dies ist ein Beleg für die erhöhte Zellstabilität der Zellen in den betreffenden Biomassen.

*Beispiel 5: Trocknung der Sulfat-reichen Biomasse aus Beispiel 1 zwecks Futtermittelherstellung*

Die Biomasse aus Beispiel 1, die im Sulfat-reichen Medium 4 erhalten wurde, wurde zwecks Herstellung von Futtermitteln einer zweistufigen Trocknung unterzogen: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein Puder mit einer Trockenmasse von mehr als 95 Gew.-% erhalten.

*Beispiel 6: Herstellung eines Futtermittels durch Extrusion*

Es wurden die in Tabelle 3 dargestellten Futtermittel-Mischungen hergestellt. Neben der erfindungsgemäß einzusetzenden Biomasse gemäß Beispiel 5 wurden zwei weitere im Handel erhältliche Labyrinthulea-Biomassen sowie Fischöl als momentan noch übliche Quelle für omega-3-Fettsäuren zum Vergleich getestet.

Die Herstellung der Futtermittel-Mischungen erfolgte jeweils durch Vermischung der Komponenten - mit Ausnahme der Öle - unter Verwendung eines Doppelhelix-Mixers (Modell 500L, TGC Extrusion, Frankreich). Die so erhaltenen Mischungen wurden anschließend unter Verwendung einer Hammermühle (Modell SH1, Hosokawa-Alpine, Deutschland) auf Teilchengrößen unterhalb 250 μm zerkleinert.

Tabelle 2: In der Extrusion eingesetzte Futtermittelzusammensetzungen (Angaben in Gew.-%)

| Inhaltsstoff | M1 | M2 | M3 | M4 |
|---|---|---|---|---|
| Fischmehl | 10,00 | 10,00 | 10,00 | 10,00 |
| Sojaprotein-Konzentrat | 23,10 | 23,20 | 23,10 | 20,27 |

(fortgesetzt)

| Inhaltsstoff | M1 | M2 | M3 | M4 |
|---|---|---|---|---|
| Erbsenprotein-Konzentrat | 15,00 | 15,00 | 15,00 | 15,00 |
| Weizen gluten | 9,90 | 9,90 | 9,90 | 9,90 |
| Weizenmehl | 18,12 | 10,82 | 10,55 | 16,46 |
| Fischöl | 10,00 | -- | -- | -- |
| Biomasse aus Beispiel 1 | -- | 16,00 | -- | -- |
| Handelsübliche Biomasse 1 | -- | -- | 16,74 | -- |
| Handelsübliche Biomasse 2 | -- | -- | -- | 13,52 |
| Rapsöl | 10,00 | 11,00 | 11,00 | 11,00 |
| Vitamin/Mineral-Prämix | 1,00 | 1,00 | 1,00 | 1,00 |
| DCP | 2,00 | 2,00 | 2,00 | 2,00 |
| Yttriumoxid | 0,03 | 0,03 | 0,03 | 0,03 |
| DL-Methionin | 0,35 | 0,36 | 0,33 | 0,33 |
| Aquavi-Lys | 0,17 | 0,35 | 0,08 | 0,19 |
| Tryp-Amino | 0,09 | 0,09 | 0,08 | 0,09 |
| L-Histidin | 0,24 | 0,25 | 0,19 | 0,21 |

Für die Extrusion wurden jeweils 140 kg pro Futtermittel eingesetzt. Die Extrusion wurde mittels eines Doppelschneckenextruders (CLEXTRAL BC45) mit einem Schneckendurchmesser von 55,5 mm und einer maximalen Fließrate von 90-100 kg/h durchgeführt. Es wurden Pellets mit einer Größe von 4,0 mm (Durchmesser und Länge) extrudiert. Der Extruder wurde hierzu mit einem Hochgeschwindigkeits-Cutter ausgestattet, um das Produkt in die vorgesehene Pellet-Größe zu überführen.

Es wurden nun verschiedene Extrusionsparameter getestet, um herauszufinden, unter welchen Extrusions-Bedingungen eine optimale Ölbeladungskapazität des erhaltenen Extrudats erhalten werden kann. Es hat sich hierbei überraschenderweise herausgestellt, dass eine optimale Ölbeladungskapazität mit einem sehr niedrigen Energieeintrag erreicht werden kann. Der Energieeintrag war hierbei deutlich geringer als bei der Verwendung von Fischöl. Weiterhin war der optimale Energieeitrag bei einer erfindungsgemäß vorzugsweise zu verwendenden Algen-Biomasse nochmal deutlich geringer als bei handelsüblichen Algen-Biomassen. Die Ergebnisse sind in Tabelle 3 dargestellt.

Tabelle 3: Energieeintrage zur Herstellung von Pellets mit der gewünschten Ölbeladungskapazität

| Diät | Barrel 1 Temp (°C) | Barrel 2 Temp (°C) | Feeder-Rate (kg/h) | Drehgeschwindigkeit (rpm) | Wassermenge (0-10) | Stromstärke (A) | SME (Wh/kg) |
|---|---|---|---|---|---|---|---|
| M1 | 31 | 116-118 | 112 | 215 | 9 | 11 | 34,6 |
| M2 | 32 | 98-104 | 141 | 253 | 5 | 7 | 20,6 |
| M3 | 32 | 97-102 | 136 | 255 | 5 | 8 | 24,6 |
| M4 | 31 | 99-107 | 133 | 253 | 5 | 8 | 24,9 |

Bei der Größe "SME" handelt es sich hierbei um die spezifische mechanische Energie. Diese wird wie folgt berechnet:

$$\text{SME (W h/kg)} = \frac{U \times I \times \cos\Phi \, \frac{\text{Test SS}}{\text{Max SS}}}{Qs}$$

mit

U: Arbeitsspannung des Motors (vorliegend 460 V)

I: Stromstärke des Motors (A)

cos Φ: theoretische Leistung des Extrudermotors (vorliegend 0,95)

Test SS: Test-Geschwindigkeit (rpm) der rotierenden Schnecken

Max SS: Maximal-Geschwindigkeit (267 rpm) der rotierenden Schnecken

Qs: Einlass-Fließrate des Futterbreis (kg/h)

Nach der Extrusion wurde das Extrudat in einem vibrierenden Fließbett-Trockner (Modell DR100, TGC Extrusion, Frankreich) getrocknet.

Abschließend erfolgte nach dem Abkühlen des Extrudats eine Öl-Beschichtung durch Vakuumbeschichtung (Vakuumbeschichter PG-10VCLAB, Dinnisen, Niederlande).

Beispiel 7: Ermittlung der Abriebfestigkeit und Wasserstabilität der Futtermittel aus Beispiel 6

Die Abriebfestigkeit wurde auf folgende Weise ermittelt: Das getrocknete Extrusionsprodukt wurde vor der Beladung mit Öl einer mechanischen Belastung unter Verwendung des Holmen Pellet-Tester (Borregaard Lignotech, Hull, UK) ausgesetzt. Vor Durchführung des Tests wurden die Proben gesiebt, um möglicherweise anhaftende Feinteilchen zu entfernen. Die aufbereiteten Proben (100 g) wurden anschließend in den Pellet-Tester eingeführt unter Verwendung eines 2,5 mm-Filtersiebs. Die Pellets wurden anschließend mit hoher Luftgeschwindigkeit für 30 Sekunden durch ein Röhrchen befördert, das rechtwinklige Rohrbogen aufwies. Anschließend wurde der Abrieb durch Wiegen bestimmt. Die Abriebfestigkeit wurde als PDI (Pellet Durability Index) angegeben, definiert als die Menge an auf dem Filtersieb verbliebener Probe in Prozent. Der Test wurde jeweils mit drei Proben durchgeführt und anschließend der Mittelwert gebildet.

Die Wasserstabilität wurde mit den ölbeladenen Proben durchgeführt. Die Methode wurde im Wesentlichen ausgeführt wie durch Baeverfjord et al. (2006; Aquaculture 261, 1335-1345) beschrieben, mit geringen Modifikationen. 10 g-Proben wurden in metallische Infusions-Körbe mit einer Maschenweite von 0,3 mm gegeben. Die Infusions-Körbe wurden anschließend in eine Plastik-Wanne mit Wasser gegeben, so dass die Proben vollständig mit Wasser bedeckt waren. Die Wanne wurde anschließend für 30 Minuten einer Schüttelagitation von 30 Schütteleinheiten pro Minute ausgesetzt. Danach wurden die Proben vorsichtig mit Löschpapier getrocknet und anschließend gewogen bevor und nachdem sie einer Ofen-Trocknung bei einer Temperatur von 105 °C für 24 Stunden unterzogen worden waren. Die Wasserstabilität wurde berechnet als die Differenz des Trockengewichts der Probe vor und nach der Inkubation in Wasser und in Prozent des Trockengewichts der eingesetzten Probe vor der Inkubation mit Wasser angegeben.

Die Ergebnisse sind in der folgenden Tabelle 4 dargestellt.

| Probe | M1 | M2 | M3 | M4 |
|---|---|---|---|---|
| Abriebfestigkeit [%] | 90,0 | 93,3 | 88,3 | 85,2 |
| Wasserstabilität [%] | 95,7 | 98,5 | 93,8 | 90,2 |

Es ist zu erkennen, dass ein erfindungsgemäßes Futtermittel, das eine erfindungsgemäße Biomasse enthält, eine deutlich höhere Abriebfestigkeit und Wasserstabilität aufweist als Futtermittel, die eine auf dem Markt erhältliche Labyrinthulea-Biomasse oder Fischöl als Quelle für omega-3-Fettsäuren enthalten.

Beispiel 8: Herstellung von Futtermittel für Fütterungsversuche

Es wurden durch Extrusion der Biomasse aus Beispiel 5 jeweils Futtermittel hergestellt, die, bezogen auf die Trockenmasse, 42,5 Gew.-% Gesamtprotein und 24 Gew.-% Gesamtlipid enthalten sowie eine Pellet-Größe von 3 mm aufweisen.

Es wurden insgesamt drei verschiedene Futtermittel-Rezepturen hergestellt (Diät 1, 2 und 3). Die Kontroll-Rezeptur "Diät 1" enthielt 11,0 Gew.-% Fischöl. In der Rezeptur "Diät 2" wurde das Fischöl teilweise (ca. 50 %) durch Aurantiochytrien-Biomasse ersetzt, indem 9,1 Gew.-% Biomasse zugefügt wurden und dafür die Menge an Fischöl auf 5,5 Gew.-% reduziert wurde. In der Rezeptur "Diät 3" wurde das Fischöl vollständig durch Aurantiochytrien-Biomasse ersetzt, indem 16 Gew.-% Biomasse zugegeben wurden und gleichzeitig die Menge an Rapsöl von 8,2 auf 9,9 Gew.-% erhöht wurde. Differenzen im Gesamtgewicht wurden durch die Menge an zugesetztem Weizen ausgeglichen.

Die einzelnen Bestandteile des Futtermittels sind in der folgenden Tabelle dargestellt.

Tabelle 5: Bei der Anzucht verwendete Rezepturen

| Komponenten (g kg$^{-1}$) | Diät 1 | Diät 2 | Diät 3 |
|---|---|---|---|
| Aurantiochytrim-Biomasse | 0,0 | 91,6 | 160,0 |
| SPC | 229,0 | 229,0 | 229,0 |
| Fischmehl | 150,0 | 150,0 | 150,0 |
| Weizen | 147,6 | 111,0 | 80,5 |
| Fischöl | 110,0 | 55,0 | 0,0 |
| Weizen gluten | 100,0 | 100,0 | 100,0 |
| Erbsenprotein-Konzentrat | 100,0 | 100,0 | 100,0 |
| Rapsöl | 82,0 | 82,0 | 99,1 |
| Mononatriumphosphat | 20,0 | 20,0 | 20,0 |
| Vitamin-Mischung | 20,0 | 20,0 | 20,0 |
| Sojalecithin | 10,0 | 10,0 | 10,0 |
| L-Lysin (50 Gew.-%) | 10,0 | 10,0 | 10,0 |
| Betafine | 9,4 | 9,4 | 9,4 |
| Mineralien-Mischung | 5,2 | 5,2 | 5,2 |
| L-Histidin (98 Gew.-%) | 4,2 | 4,2 | 4,2 |
| DL-Methionin (99 Gew.-%) | 2,0 | 2,0 | 2,0 |
| Carop. Pink (10 Gew.-%) | 0,50 | 0,50 | 0,50 |
| Yttriumoxid | 0,10 | 0,10 | 0,10 |

Die einzelnen Komponenten wurden - mit Ausnahme der Öle - innig miteinander vermischt und anschließend unter Verwendung eines Doppelschnecken-Extruders (Wenger TX 52, Wenger, USA) durch Anwendung einer Austrittsdüse mit einem Durchmesser von 2 mm ein Extrudat hergestellt. Die Extrudate wurden ca. 1 Stunde in einem Karussell-Trockner (Paul Klöckner, Verfahrenstechnik GmbH, Deutschland) bei 65°C auf einen Wassergehalt von 7 bis 8 Gew.-% getrocknet. Anschließend wurden die Extrudate über Nacht bei Raumtemperatur getrocknet, bevor die Öle durch Vakuum-Beschichtung (Dinnissen, Sevenum, Niederlande) aufgetragen wurden.

*Beispiel 9: Fütterungsexperimente mit den Rezepturen aus Beispiel 8*

Zur Durchführung der Fütterungsexperimente wurden jeweils drei Behälter, die junge Lachse mit einem Gewicht von durchschnittlich 83,6 g bei einem Gesamtgewicht an Lachs von 4 kg pro Behälter enthielten, mit jeder dieser Rezepturen für insgesamt 12 Wochen gefüttert.

Das Gesamtgewicht an Lachs pro Behälter nahm in dieser Zeit von 4 kg auf 15-17 kg pro Behälter zu. Die Fische konsumierten hierbei 8 bis 11 kg Futter pro Tank, was einer Futter-Konversionsrate (FCR) von 0,8 bis 0,9 kg Futter pro kg Fisch entspricht.

Die Ergebnisse der Fütterungsexperimente sind in der nachfolgenden Tabelle dargestellt.

Tabelle 6: Gewichtszuwachs der Fische in Abhängigkeit von der Diät

| Diät | Endgewicht [g] |
|---|---|
| 1 | 331 |

(fortgesetzt)

| Diät | Endgewicht [g] |
|------|----------------|
| 2 | 362 |
| 3 | 339 |

Insgesamt wurde festgestellt, dass sowohl bei vollständigem als auch bei teilweisem Ersatz des Fischöls durch eine erfindungsgemäße Aurantiochytrien-Biomasse eine Steigerung des Wachstums der Lachse erzielt werden konnte.

Interessanterweise wurde bei teilweisem Ersatz des Fischöls durch die Aurantiochytrien-Biomasse ein höheres Wachstum der Lachse erzielt als bei vollständigem Ersatz durch die Aurantiochytrien-Biomasse.

Es wurde hierbei festgestellt, dass die Fische, die mit der Kontroll-Rezeptur Diät 1 gefüttert wurden, mit einem Endgewicht von durchschnittlich 331 g ein deutlich geringeres Endgewicht aufwiesen als die Fische, die mit den Rezepturen Diät 1 oder 2 gefüttert wurden. Am besten schnitten hierbei die Fische ab, die mit der Rezeptur Diät 2 gefüttert wurden: Sie erreichten ein deutlich erhöhtes Endgewicht von durchschnittlich 362 g.

*Beispiel 10: Fettsäureverwertung durch den Fisch*

Zur Ermittlung der Fettsäureverwertung erfolgte der Lipidnachweis unter Verwendung der Bligh & Dryer-Extraktionsmethode und anschließender Fettsäureanalyse gemäß AOCS Ce 1b-89. Es wurden sowohl Muskel-Proben als auch Gesamt-Lachsproben analysiert. Es wurden hierbei die in den folgenden Tabellen dargestellten Ergebnisse erhalten (angegeben ist jeweils die Menge an ermittelten Fetten am Start bzw. nach Ende der Diät in Gramm jeweils in Bezug auf 100 g Gesamtfett).

Tabelle 7: Fettsäureprofile von Lachsmuskel-Proben in Abhängigkeit von der Diät

| Diät | PUFAs | Omega-3-Fettsäuren | DHA |
|------|-------|--------------------|----|
| Start | 41,6 | 32,7 | 22,1 |
| 1 | 31,8 | 19,0 | 10,1 |
| 2 | 35,3 | 21,7 | 14,2 |
| 3 | 38,0 | 22,8 | 16,4 |

Tabelle 8: Fettsäureprofile von Gesamtlachs-Proben in Abhängigkeit von der Diät

| Diät | PUFAs | Omega-3-Fettsäuren | DHA |
|------|-------|--------------------|----|
| Start | 32,5 | 22,4 | 12,5 |
| 1 | 29,9 | 17,2 | 9,0 |
| 2 | 32,6 | 18,8 | 11,5 |
| 3 | 35,2 | 19,6 | 13,1 |

Es ist zu beobachten, dass bereits bei teilweisem Ersatz des Fischöls durch eine erfindungsgemäße Aurantiochytrien-Biomasse eine deutliche Steigerung des Gehalts an PUFAs, omega-3-Fettsäuren und DHA erreicht werden konnte. Bei vollständigem Ersatz des Fischöls durch die Aurantiochytrien-Biomasse fällt die Steigerung des Gehalts an PUFAs entsprechend höher aus.

*Beispiel 11: Bestimmung des Fettgehalts der Lachsleber*

Jeweils 3 junge Lachse wurden für jeweils 9 Wochen mit den unterschiedlichen Rezepturen Diät 1, 2 und 3 gefüttert und anschließend zur Bestimmung des Fettgehalts die Leber der Lachse entnommen. Die Fettextraktion erfolgte gemäß der Methode von Folch (1957; J. Biol. Chem., 226 (1), 497-509). Der Fettgehalt wurde anschließend durch eine gravimetrische Methode bestimmt.

Es stellte sich heraus, dass aufgrund der Anwesenheit der erfindungsgemäßen Biomasse im Vergleich zur Fütterung ohne Biomasse der Fettgehalt in der Leber von 8 Gew.-% signifikant auf 4-5 Gew.-% reduziert werden konnte.

Die Anlagerung von Fett in der Leber gilt als ein Anzeichen für ein Ungleichgewicht des Nahrungsstoffwechsels und insbesondere auch als Hinweis auf oxidativen Stress. Die deutliche Reduktion des Fettanteils in der Leber ist somit ein deutlicher Hinweis für die Reduktion von Stress und somit für die Verbesserung des Gesundheitszustands der Lachse.

**Patentansprüche**

1. PUFAs enthaltende Biomasse, **dadurch gekennzeichnet, dass** sie einen Sulfat-Gehalt, bezogen auf die Trockenmasse, von 25 bis 40 g/kg und einen Feuchtigkeitsgehalt von unter 5 Gew.-% aufweist, wobei die Biomasse Zellen des Taxons Labyrinthulomycetes enthält und der Sulfat-Gehalt durch Ermittlung des Schwefel-Gehalts mittels Elementaranalyse gemäß DIN EN ISO 11885 bestimmt wird.

2. PUFAs enthaltende Biomasse nach vorhergehendem Anspruch, **dadurch gekennzeichnet, dass** sie einen Sulfat-Gehalt, bezogen auf die Trockenmasse, von 25 bis 30 g/kg aufweist.

3. PUFAs enthaltende Biomasse nach einem der vorhergehenden Ansprüche, **dadurch gekennzeichnet, dass** es sich bei den Zellen des Taxons Labyrinthulomycetes
um solche der Familie der Thraustochytriaceae, vorzugsweise der Gattungen Althomia, Aplanochytrium, Elnia, Japonochytrium, Schizochytrium, Thraustochytrium, Aurantiochytrium, Oblongichytrium oder Ulkenia, handelt.

4. PUFAs enthaltende Biomasse nach vorhergehendem Anspruch, **dadurch gekennzeichnet, dass** es sich bei den Mikroorganismen um Zellen der Gattung Aurantiochytrium, vor allem um solche der Spezies Aurantiochytrium limacinum, insbesondere um solche des Stamms Aurantiochytrium limacinum SR21 (IFO 32693), handelt.

5. Verfahren zur Herstellung eines Futtermittels, **dadurch gekennzeichnet, dass** eine PUFAs enthaltende Biomasse nach einem der vorhergehenden Ansprüche mit weiteren Futtermittelkomponenten vermischt und anschließend zu einem Futtermittel extrudiert wird und gegebenenfalls das so erhaltene Extrudat anschließend mit Öl beschichtet wird.

6. Verfahren nach vorhergehendem Anspruch, **dadurch gekennzeichnet, dass** die bei der Extrusion eingesetzte Zusammensetzung folgende Eigenschaften aufweist:

   a) einen Gesamt-Proteingehalt von 33 bis 67 Gew.-%, vorzugsweise 39 bis 61 Gew.-%, insbesondere 44 bis 55 Gew.-%;
   b) einen Gesamt-Fettgehalt von 5 bis 25 Gew.-%, vorzugsweise 8 bis 22 Gew.-%, insbesondere 10 bis 20 Gew.-%, vor allem 12 bis 18 Gew.-%;
   c) einen Gesamt-Stärkegehalt von maximal 25 Gew.-%, insbesondere maximal 20 Gew.-%, vorzugsweise 6 bis 17 Gew.-%, besonders bevorzugt 8 bis 14 Gew.-%;
   d) einen Gehalt an Biomasse, insbesondere Labyrinthulea-Biomasse, bevorzugt Thraustochytriaceae-Biomasse, von 2 bis 24 Gew.-%, vorzugsweise 4 bis 22 Gew.-%, insbesondere 9 bis 20 Gew.-%, vor allem 11 bis 18 Gew.-%;
   e) vorzugsweise einen Gehalt an polyungesättigten Fettsäuren (PUFAs) von 0,8 bis 8 Gew.-%, vorzugsweise 1,2 bis 6 Gew.-%, insbesondere 1,4 bis 5 Gew.-%, vor allem 1,5 bis 4 Gew.-%;
   f) vorzugsweise einen Gehalt an omega-3-Fettsäuren von 0,8 bis 8 Gew.-%, vorzugsweise 1,2 bis 6 Gew.-%, insbesondere 1,4 bis 5 Gew.-%, vor allem 1,5 bis 4 Gew.-%;
   g) vorzugsweise Gehalt an DHA von 0,1 bis 4,0 Gew.-%, vorzugsweise 0,25 bis 3,0 Gew.-%, insbesondere 0,5 bis 2,8 Gew.-%, vor allem 0,8 bis 2,5 Gew.-%, insbesondere 1,0 bis 2,0 Gew.-%.

7. Verfahren nach einem der vorhergehenden Ansprüchen, **dadurch gekennzeichnet, dass** die Extrusion bei einem Energieeintrag von 12 - 28 Wh/kg, insbesondere 14 - 26 Wh/kg, besonders bevorzugt 16 - 24 Wh/kg, durchgeführt wird.

8. Verfahren nach einem der vorhergehenden Ansprüchen, **dadurch gekennzeichnet, dass** nach der Extrusion und gegebenenfalls Trocknung des Extrudats eine Beschichtung des Extrudats mit Öl, insbesondere pflanzlichem Öl, vorzugsweise in einer Menge von, in Bezug auf das finale Produkt, 3 bis 17 Gew.-%, insbesondere 5 bis 15 Gew.-%, erfolgt.

9. Futtermittel, **dadurch gekennzeichnet, dass** es eine PUFAs enthaltende Biomasse nach einem der Ansprüche 1 bis 4 enthält.

**10.** Futtermittel gemäß Anspruch 9, **dadurch gekennzeichnet, dass** es folgende Eigenschaften aufweist:

a) einen Gesamt-Proteingehalt von 30 bis 60 Gew.-%, vorzugsweise 35 bis 55 Gew.-%, insbesondere 40 bis 50 Gew.-%;

b) einen Gesamt-Fettgehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

d) einen Gehalt an Biomasse, insbesondere an Labyrinthulea-Biomasse, vorzugsweise an Thraustochytriaceae-Biomasse, von 2 bis 22 Gew.-%, vorzugsweise 4 bis 20 Gew.-%, insbesondere 8 bis 18 Gew.-%, vor allem 10 bis 16 Gew.-%;

e) vorzugsweise einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;

f) vorzugsweise einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

g) vorzugsweise einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

**11.** Verfahren zur Anzucht von Tieren, insbesondere Fischen, bei welchem ein Futtermittel nach einem der vorhergehenden Ansprüche zum Einsatz kommt.

**Claims**

**1.** PUFAs-containing biomass, **characterized in that** it has a sulphate content, based on the dry mass, of 25 to 40 g/kg and a moisture content of below 5% by weight, wherein the biomass contains cells of the taxon Labyrinthulomycetes and the sulphate content is determined by ascertaining the sulphur content by means of elemental analysis in accordance with DIN EN ISO 11885.

**2.** PUFAs-containing biomass according to the preceding claim, **characterized in that** it has a sulphate content, based on the dry mass, of 25 to 30 g/kg.

**3.** PUFAs-containing biomass according to any of the preceding claims, **characterized in that** the cells of the taxon Labyrinthulomycetes are those of the family of the Thraustochytriaceae, preferably of the genera Althomia, Aplanochytrium, Elnia, Japonochytrium, Schizochytrium, Thraustochytrium, Aurantiochytrium, Oblongichytrium or Ulkenia.

**4.** PUFAs-containing biomass according to the preceding claim, **characterized in that** the microorganisms are cells of the genus Aurantiochytrium, above all those of the species Aurantiochytrium limacinum, in particular those of the strain Aurantiochytrium limacinum SR21 (IFO 32693).

**5.** Process for producing a feedstuff, **characterized in that** a PUFAs-containing biomass according to any of the preceding claims is mixed with further feedstuff components and then extruded to form a feedstuff and, optionally, the extrudate thus obtained is then coated with oil.

**6.** Process according to the preceding claim, **characterized in that** the composition used for extrusion has the following properties:

a) a total protein content of 33 to 67% by weight, preferably 39 to 61% by weight, in particular 44 to 55% by weight;

b) a total fat content of 5 to 25% by weight, preferably 8 to 22% by weight, in particular 10 to 20% by weight, above all 12 to 18% by weight;

c) a total starch content of at most 25% by weight, in particular at most 20% by weight, preferably 6 to 17% by weight, especially preferably 8 to 14% by weight;

d) a content of biomass, in particular Labyrinthulea biomass, preferably Thraustochytriaceae biomass, of 2 to 24% by weight, preferably 4 to 22% by weight, in particular 9 to 20% by weight, above all 11 to 18% by weight;

e) preferably a content of polyunsaturated fatty acids (PUFAs) of 0.8 to 8% by weight, preferably 1.2 to 6% by weight, in particular 1.4 to 5% by weight, above all 1.5 to 4% by weight;

f) preferably a content of omega-3 fatty acids of 0.8 to 8% by weight, preferably 1.2 to 6% by weight, in particular 1.4 to 5% by weight, above all 1.5 to 4% by weight;

g) preferably a content of DHA of 0.1 to 4.0% by weight, preferably 0.25 to 3.0% by weight, in particular 0.5 to 2.8% by weight, above all 0.8 to 2.5% by weight, in particular 1.0 to 2.0% by weight.

7. Process according to any of the preceding claims, **characterized in that** the extrusion is done at an energy input of 12 - 28 Wh/kg, in particular 14 - 26 Wh/kg, especially preferably 16 - 24 Wh/kg.

8. Process according to any of the preceding claims, **characterized in that** the extrusion and optionally drying of the extrudate is followed by coating of the extrudate with oil, in particular vegetable oil, preferably in an amount of, with regard to the final product, 3 to 17% by weight, in particular 5 to 15% by weight.

9. Feedstuff, **characterized in that** it contains a PUFAs-containing biomass according to any of Claims 1 to 4.

10. Feedstuff according to Claim 9, **characterized in that** it has the following properties:

a) a total protein content of 30 to 60% by weight, preferably 35 to 55% by weight, in particular 40 to 50% by weight;
b) a total fat content of 15 to 35% by weight, preferably 18 to 32% by weight, in particular 20 to 30% by weight, above all 22 to 28% by weight;
c) a total starch content of at most 25% by weight, in particular at most 20% by weight, preferably 5 to 15% by weight, especially preferably 7 to 13% by weight;
d) a content of biomass, in particular Labyrinthulea biomass, preferably Thraustochytriaceae biomass, of 2 to 22% by weight, preferably 4 to 20% by weight, in particular 8 to 18% by weight, above all 10 to 16% by weight;
e) preferably a content of polyunsaturated fatty acids (PUFAs) of 2 to 12% by weight, preferably 3 to 10% by weight, in particular 4 to 9% by weight, above all 5 to 8% by weight;
f) preferably a content of omega-3 fatty acids of 1 to 6% by weight, preferably 1.5 to 5% by weight, in particular 2 to 4.5% by weight, above all 2.5 to 4% by weight;
g) preferably a content of DHA of 0.5 to 3% by weight, preferably 0.8 to 2.5% by weight, in particular 1 to 2.5% by weight, above all 1.2 to 2.2% by weight, in particular 1.2 to 2.0% by weight.

11. Method for farming animals, in particular fish, in which a feedstuff according to any of the preceding claims is used.

## Revendications

1. Biomasse contenant des AGPI, **caractérisée en ce qu'**elle présente une teneur en sulfates, rapportée à la matière sèche, de 25 à 40 g/kg et une teneur en humidité inférieure à 5 % en poids, la biomasse contenant des cellules du taxon Labyrinthulomycetes, et la teneur en sulfates étant déterminée par détermination de la teneur en soufre par une analyse élémentaire selon DIN EN ISO 11885.

2. Biomasse contenant des AGPI selon la revendication précédente, **caractérisée en ce qu'**elle présente une teneur en sulfates, rapportée à la matière sèche, de 25 à 30 g/kg.

3. Biomasse contenant des AGPI selon l'une quelconque des revendications précédentes, **caractérisée en ce que**, pour ce qui concerne les cellules du taxon Labyrinthulomycetes, il s'agit de celles de la famille des Thraustochytriaceae, de préférence des genres Althomia, Aplanochytrium, Elnia, Japonochytrium, Schizochytrium, Thraustochytrium, Aurantiochytrium, Oblongichytrium ou Ulkenia.

4. Biomasse contenant des AGPI selon la revendication précédente, **caractérisée en ce que**, pour ce qui concerne les microorganismes, il s'agit de cellules du genre Aurantiochytrium, surtout de celles de l'espèce Aurantiochytrium limacinum, en particulier de celles de la souche Aurantiochytrium limacinum SR21 (IFO 32693).

5. Procédé de fabrication d'un aliment pour animaux, **caractérisé en ce qu'**on mélange une biomasse contenant des AGPI selon l'une des revendications précédentes à d'autres constituants d'aliments pour animaux, puis on l'extrude pour obtenir un aliment pour animaux, et éventuellement on enduit ensuite d'huile l'extrudat ainsi obtenu.

6. Procédé selon la revendication précédente, **caractérisé en ce que** la composition utilisée pour l'extrusion présente les propriétés suivantes :

a) une teneur totale en protéines de 33 à 67 % en poids, de préférence de 39 à 61 % en poids, en particulier de 44 à 55 % en poids ;

b) une teneur totale en lipides de 5 à 25 % en poids, de préférence de 8 à 22 % en poids, en particulier de 10 à 20 % en poids, surtout de 12 à 18 % en poids ;

c) une teneur totale en amidon au maximum de 25 % en poids, en particulier au maximum de 20 % en poids, de préférence de 6 à 17 % en poids, d'une manière particulièrement préférée de 8 à 14 % en poids ;

d) une teneur en biomasse, en particulier en biomasse de Labyrinthulea, de préférence en biomasse de Thraustochytriaceae, de 2 à 24 % en poids, de préférence de 4 à 22 % en poids, en particulier de 9 à 20 % en poids, surtout de 11 à 18 % en poids ;

e) de préférence une teneur en acides gras polyinsaturés (AGPI) de 0,8 à 8 % en poids, de préférence de 1,2 à 6 % en poids, en particulier de 1,4 à 5 % en poids, surtout de 1,5 à 4 % en poids ;

f) de préférence une teneur en acides gras oméga-3 de 0,8 à 8 % en poids, de préférence de 1,2 à 6 % en poids, en particulier de 1,4 à 5 % en poids, surtout de 1,5 à 4 % en poids ;

g) de préférence une teneur en DHA de 0,1 à 4,0 % en poids, de préférence de 0,25 à 3,0 % en poids, en particulier de 0,5 à 2,8 % en poids, surtout de 0,8 à 2,5 % en poids, en particulier de 1,0 à 2,0 % en poids.

**7.** Procédé selon l'une des revendications précédentes, **caractérisé en ce que** l'extrusion est mise en œuvre avec un apport d'énergie de 12 à 28 Wh/kg, en particulier de 14 à 26 Wh/kg, d'une manière particulièrement préférée de 16 à 24 Wh/kg.

**8.** Procédé selon l'une des revendications précédentes, **caractérisé en ce que**, après l'extrusion et éventuellement après le séchage de l'extrudat, on procède à une enduction de l'extrudat avec une huile, en particulier une huile végétale, de préférence en une quantité, rapportée au produit final, de 3 à 17 % en poids, en particulier de 5 à 15 % en poids.

**9.** Aliment pour animaux, **caractérisé en ce qu'**il contient une biomasse contenant des AGPI selon l'une des revendications 1 à 4.

**10.** Aliment pour animaux selon la revendication 9, **caractérisé en ce qu'**il présente les propriétés suivantes :

a) une teneur totale en protéines de 30 à 60 % en poids, de préférence de 35 à 55 % en poids, en particulier de 40 à 50 % en poids ;

b) une teneur totale en lipides de 15 à 35 % en poids, de préférence de 18 à 32 % en poids, en particulier de 20 à 30 % en poids, surtout de 22 à 28 % en poids ;

c) une teneur totale en amidon au maximum de 25 % en poids, en particulier au maximum de 20 % en poids, de préférence de 5 à 15 % en poids, d'une manière particulièrement préférée de 7 à 13 % en poids ;

d) une teneur en biomasse, en particulier en biomasse de Labyrinthulea, de préférence en biomassse de Thraustochytriaceae, de 2 à 22 % en poids, de préférence de 4 à 20 % en poids, en particulier de 8 à 18 % en poids, surtout de 10 à 16 % en poids ;

e) de préférence une teneur en acides gras polyinsaturés (AGPI) de 2 à 12 % en poids, de préférence de 3 à 10 % en poids, en particulier de 4 à 9 % en poids, surtout de 5 à 8 % en poids ;

f) de préférence une teneur en acides gras oméga-3 de 1 à 6 % en poids, de préférence de 1,5 à 5 % en poids, en particulier de 2 à 4,5 % en poids, surtout de 2,5 à 4 % en poids ;

g) de préférence une teneur en DHA de 0,5 à 3 % en poids, de préférence de 0,8 à 2,5 % en poids, en particulier de 1 à 2,5 % en poids, surtout de 1,2 à 2,2 % en poids, en particulier de 1,2 à 2,0 % en poids.

**11.** Procédé pour l'élevage d'animaux, en particulier de poissons, dans lequel on utilise un aliment pour animaux selon l'une des revendications précédentes.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 05154510 A
- WO 9107498 A
- WO 9408467 A
- WO 9737032 A
- WO 9736996 A
- WO 0154510 A
- EP 13176661
- EP 13187631

**In der Beschreibung aufgeführte Nicht-Patentliteratur**

- **R.H. MÜLLER ; R. SCHUHMANN.** Teilchengrößenmessung in der Laborpraxis. *Wissenschaftliche Verlagsgesellschaft Stuttgart,* 1996
- **M. RHODES.** Introduction to Particle Technology. Verlag Wiley & Sons, 1998
- **BAEVERFJORD et al.** *Aquaculture,* 2006, vol. 261, 1335-1345
- *J. Biol. Chem.,* 1957, vol. 226 (1), 497-509